# EUROPEAN PATENT SPECIFICATION

(11) **EP 3 397 557 B1**
(45) Date of publication and mention of the grant of the patent: **20.01.2021**
(21) Application number: 16823353.4
(22) Date of filing: 30.12.2016
(51) Int. Cl.: B65B 11/32, A61F 13/20, B65B 19/00, B65B 47/06, B65B 49/12

(54) **METHOD OF FORMING PRIMARY PACKAGE TUBE FOR TAMPONS AND THE CORRESPONDING APPARATUS**
VERFAHREN ZUR HERSTELLUNG EINES PRIMÄRVERPACKUNGSROHRS FÜR TAMPONS UND DIE ENTSPRECHENDE VORRICHTUNG
PROCÉDÉ DE FORMATION D'UN TUBE D'EMBALLAGE PRIMAIRE POUR TAMPONS ET L'APPAREIL CORRESPONDANT

(30) Priority: 31.12.2015 US 201562273775 P
(43) Date of publication of application: 07.11.2018
(73) Proprietor: Johnson and Johnson GmbH, 41470 Neuss (DE)
(72) Inventor: CANKAR, Thomas, 42289 Wuppertal (DE); KLAR, Markus, 42289 Wuppertal (DE); BERGERMANN, Martin, 42289 Wuppertal (DE); ARNESEN, Jens-Petter, 51120 Sezanne (FR); ANGER, Markus, 42289 Wuppertal (DE)
(74) Representative: Kirsch, Susan Edith
(86) International application number: PCT/IB2016/058118
(87) International publication number: WO 2017/115335

(56) References cited:
- EP-A1- 1 477 406
- GB-A- 191 500 921
- NL-A- 6 917 298
- US-A- 5 987 847

## Description

### Field of the Invention

The present invention relates to a method of packaging a tampon.

### Background of the Invention

Methods for closing one end of tubular containers are known.

US 8221371 by McNeil-PPC Inc. discloses a wrapper with a folded bottom and a process for overwrapping a tampon. The process comprises a cylindrical wrapper that projects beyond the end of a tampon. The withdrawal end is heat sealed. The insertion end may be a dome or have a flattened geometry. Jaws, preferably four jaws, contact the insertion end causing the overlap to surround the insertion end of the tampon and be sealed together. For this purpose said jaws are spring mounted and heated. A folding station folds excess of overwrap.

US 5,907,941 by Fuji Photo Film. Co. purports to disclose a wrapping apparatus for a web roll, preferably a photographic film. A sheet is being wound around said web roll. The web roll has a core that is connected to a holding member. A circular rack is fixed to a folding unit, a heating member and a cooling member. Said rack may rotate around an axis and thereby around the holding member with the fixed web roll. Now the folding unit, a heating member and a cooling member are moved radially closer to the axis in order to contact the rim of the web roll. The folding unit rotates in counter direction to the rotation of the rack and folding blades are used to obtain distinguished fold lines. Said heating member seals the fold lines, which are cooled by the cooling member.

GB 966121 purports to disclose a wrapping machine for a roll. Any cylindrical article may be used. The wrapping machine comprises a number of folder plates spaced at angular intervals around a main axis coaxial with the roll, means for causing the folder plates to move simultaneously inwardly towards the main axis and thereby to fold the tubular projecting end of the wrapping material, and means for rotating the assembly of folder plates through an angle around the main axis, thereby flattening the pleats of the wrapping material closely against the end of the roll. Preferably the folder blades are pivoted to support members that may move inward.

US 3807132 purports to disclose a wrapping machine for a "cylindrical thing", e.g. a toilet paper roll, having an axial bore. For the wrapping process it is mounted on a pushing member. A cylindrical frame rotates around the cylindrical thing and grasping members move radially inwards for the folding process. The rotation causes the folding to flatten and folding pattern.

EP 1477406 relates to a method and machine for packing a tampon, whereby the tampon is inserted into a tubular folding spindle of constant cross section; a sheet of packing material is then wrapped about the folding spindle and stabilized longitudinally to form a tubular wrapping; the folding spindle and the tubular wrapping, together with the tampon, are then parted.; and the two ends of the tubular wrapping are closed transversely about the relative tampon.

US 5987847 relates to a machine and method for wrapping cylindrical workpieces such as rolls of bathroom tissue and paper towel.

However, none of these references teach a method and apparatus for high-speed closure of a tubular plastic wrapper for use with tampons.

### Summary of the Invention

The present invention relates to high-speed methods to close tubular plastic wrappers for use with tampons. According to the invention, the process includes providing a packaging film on a mandrel in the form of a hollow tube, forming a folded surface by rotating the hollow tube and mandrel while counter-rotating a plate having a plurality of folding blades extending outwardly therefrom, to form a plurality of folds that define the folded surface, substantially perpendicular to the longitudinal axis of the hollow tube, each folding blade engaging a portion of the protruding tube portion, applying heat and pressure to the folded surface to form a closed end, inserting the tampon into the tube, and closing the first end of the tube. The packaging film material comprises at least one thermoplastic surface, and the mandrel has a first end extending from a revolving platform and a second end, distal the first end. In addition, the mandrel is capable of rotating with respect to the revolving platform on which it is mounted. In addition, the hollow tube has an overlapping longitudinal seam area having two plies of the packaging film and has a first end corresponding to the first end of the mandrel and a second end distal thereof, the second end extending beyond the second end of the mandrel to define a protruding tube portion, and a length extending from the first end to the second end; wherein the protruding tube portion has a length approximately equal to the radius of the hollow tube.

In an alternative embodiment, the folded surface is formed by rotating a plurality of folding blades perpendicular to the longitudinal axis of the hollow tube to engage the protruding tube portion to form a plurality of folds that define the folded surface, substantially perpendicular to the longitudinal axis of the hollow tube.

### Brief Description of the Drawings

Fig. 1 is a side elevation of an open wrapping tube disposed on a mandrel.
Fig. 2 is an end view of the open wrapping tube and mandrel of Fig. 1.
Fig. 3 is a plan view of a fan-like folding plate useful in the method of the present invention.
Fig. 4 is a side view of the fan-like folding plate of Fig. 3.
Fig. 5 is a perspective view of a folding blade of the folding plate of Fig. 3.
Fig. 6 is a perspective view of a revolver and ancillary equipment useful in the practice of the present invention.
Figs. 7 are close-up views of the engagement of the fan-like folding plate of Fig. 3 and the mandrel of Fig. 2 during the process of forming pleats across the second end of the wrapping tube of Fig. 2.
Figs. 9-11 are plan views of an alternative embodiment of the wrapper tube closure apparatus which is not in the scope of the present invention.
Fig. 12 is a perspective view of an alternative embodiment of the wrapper tube closure apparatus of the present invention.

### Detailed Description of the Invention

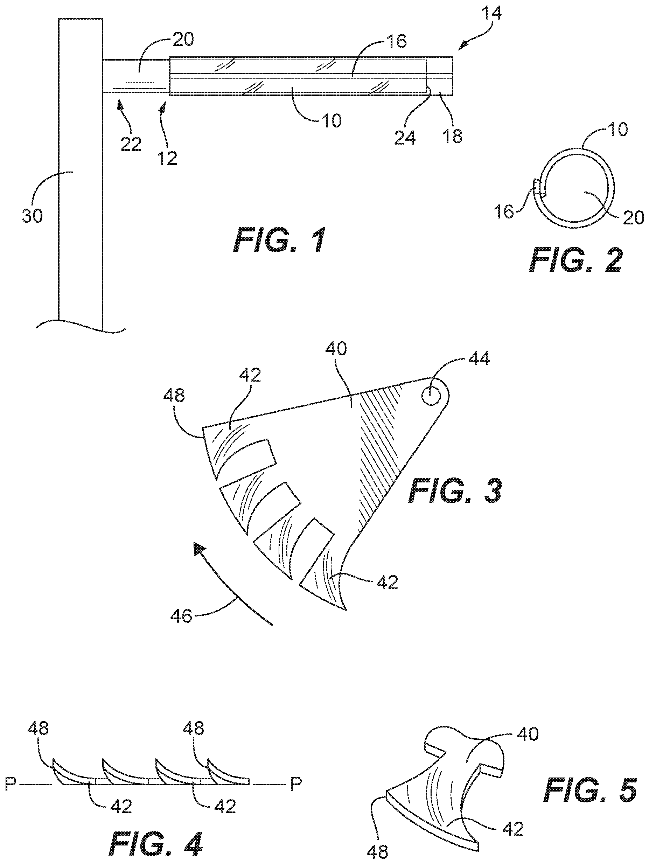

The following is a detailed description of the present invention, wherein like elements are labeled with like numerals in Figs. 1-11. Referring to Figs. 1-2, a plastic wrapping film, formed into a flexible packaging tube 10 is disposed on a mandrel 20. The flexible packaging tube 10 has a first open end 12, a second open end 14 and a longitudinal seam 16. The mandrel 20 has a first end 22 extending from a revolving platform 30 and a second end 24, distal the first end 22, and a length extending from the first end 22 to the second end 24, and is capable of rotating with respect to the revolving platform 30. The second end 14 of the flexible packaging tube 10 extends beyond the second end 24 of the mandrel 20 to define a protruding tube portion 18.

Figs. 3-5 show features of a fan-like folding plate 40 having four folding blades 42 that is rotatable about pivot 44 in the direction of arrow 46. When the folding plate 40 rotates in the direction of arrow 46, the leading edge 48 of each folding blade 42 is elevated with respect to the plane of the folding plate 40 to enable the folding blade 42 to engage the protruding portion 18 of the flexible packaging tube 10. The remainder of the folding blade 42 curves and/or slopes down to the plane "p-p" of the folding plate 40 (shown in Fig. 4). Thus, as each folding blade 42 moves across the protruding portion 18 flexible packaging tube 10, it forms a pleat across the open second end 24 of the flexible packaging tube 10. The four folding blades 42 of the embodiment of these figures would be capable of forming four pleats across the open end 24 of the flexible packaging tube 10. After these pleats are formed, they can be heated under pressure to soften the plastic wrapping film material to seal them in a manner to close the previously open second end 14 of the flexible packaging tube 10.

While four folding blades are shown in this embodiment, one of ordinary skill in the art will recognize that other numbers may be used, from three to eight or more. Preferably, the number of blades is between four and six.

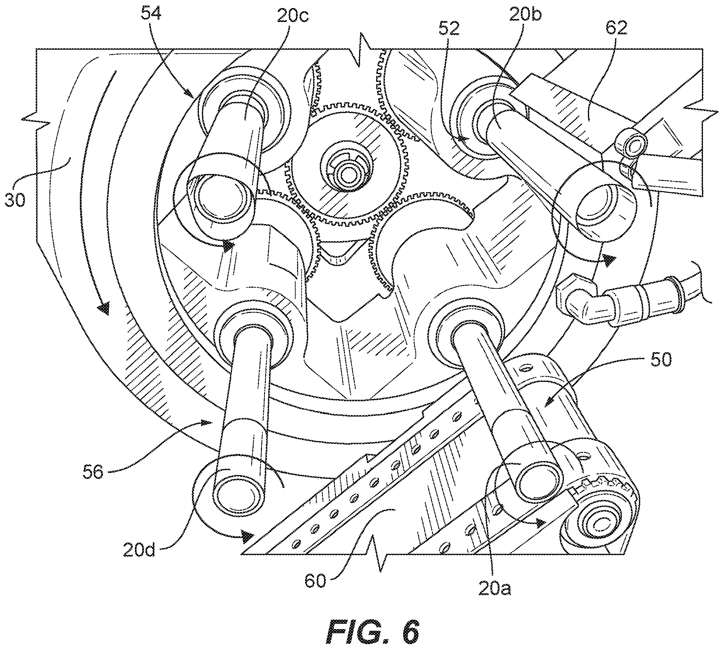

Fig. 6 shows a revolving platform 30 having mounted thereon four mandrels 20. As shown in Position 50, a first mandrel 20a engages a conveyor 60 that delivers a supply of plastic wrapping film thereto. Each mandrel 20 can be equipped with apertures and/or slots to permit vacuum pressure to be drawn therethrough to hold the plastic wrapping film in place on the mandrel. As shown in Position 52, a second mandrel 20b is engaged with a longitudinal sealing bar 62 arranged and configured to seal overlapping plies of the plastic wrapping film to form the flexible packaging tube 10. Position 54 is the tube folding apparatus (not shown in this figure) and has a third mandrel 20c with a flexible packaging tube 10 ready for engagement with the tube folding apparatus. At Position 56, a fourth mandrel 20d has transferred the flexible packaging tube 10 for further processing.

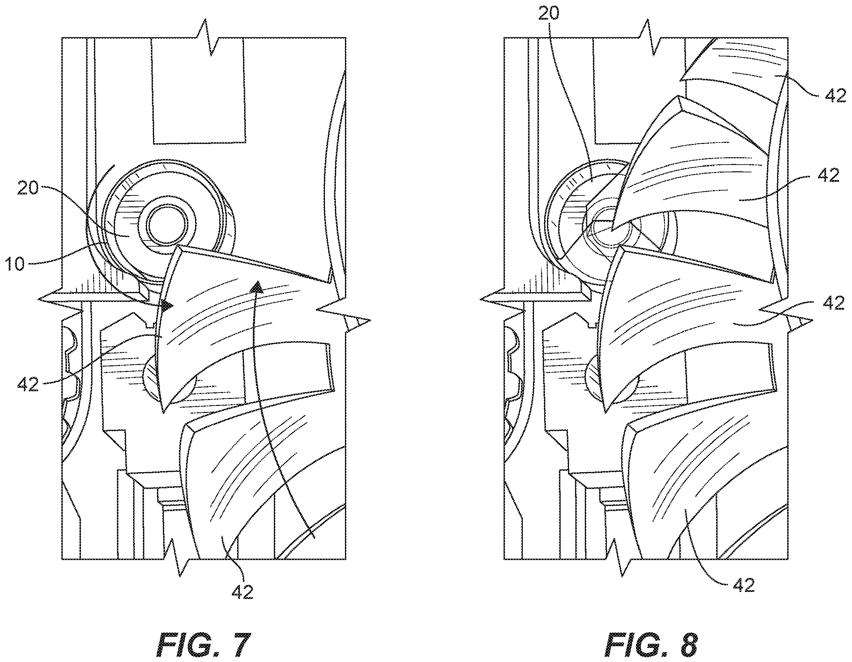

Figs. 7 and 8 show the engagement of the folding blades 42 with the protruding portion 18 of the flexible packaging tube 10. As shown in Fig. 7, the mandrel 20 rotates in a counter-clockwise direction, while the folding blades 42 rotate in a clockwise direction. Fig. 8 shows the process partially completed with two of the folding blades 42 having made a corresponding number of folds across the second open end 14.

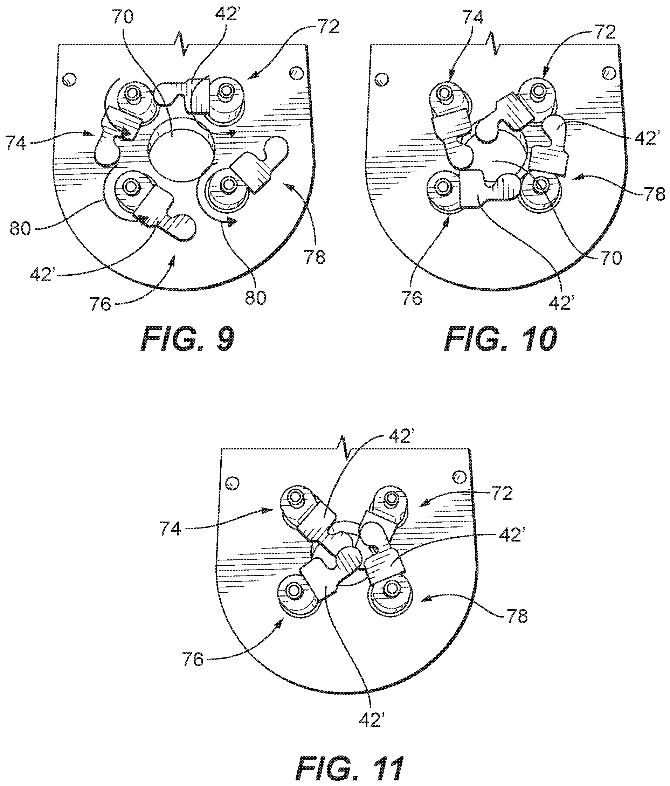

In an alternative embodiment which is not in the scope of the invention, shown in Figs. 9-11, the mandrel (not shown) remains stationary in aperture 70 while four independently rotatable folding blades 42' rotate, sequentially, e.g., starting at Position 72, followed by Position 74, Position 76, and Position 78. Fig. 9 shows the four independent folding blades 42' ready to fold in the direction of arrows 80. Fig. 10 shows the folding blades 42' corresponding to Positions 72 furthest across aperture 70 followed closely by Position 74, while the folding blade 42' corresponding to Position 76 approaches the aperture 70. Fig. 11 shows all four folding blades 42' disposed across aperture 70. After these pleats are formed, they can be heated under pressure to soften the plastic wrapping film material to seal them in a manner to close the previously open second end 14 of the flexible packaging tube 10.

While four folding blades are shown in this embodiment, one of ordinary skill in the art will recognize that other numbers may be used, from three to eight or more. Preferably, the number of blades is between four and six.

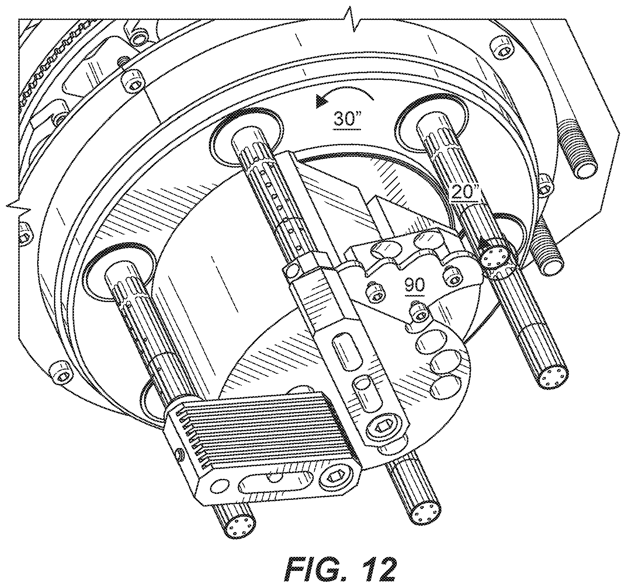

In an alternative embodiment, shown in Fig. 12, the revolving platform 30 and the mandrel 20 are rotating along a fixed wave shaped plate 90. Each wave forms an individual fold or pleat. After these pleats are formed, they are heated under pressure to soften the plastic wrapping film material to seal them in a manner to close the previously open second end (not shown) of the flexible packaging tube (not shown).

While four folding waves are shown in this embodiment, one of ordinary skill in the art will recognize that other numbers may be used, from three to eight or more. Preferably, the number of waves is between four and six.

## Claims

1. A method of packaging a tampon comprising the steps of:
a. providing a packaging film on a mandrel (20) in the form of a hollow tube (10), wherein:
i. the packaging film material comprises at least one thermoplastic surface;
ii. the mandrel (20) has a first end (22) extending from a revolving platform (30) and a second end (24), distal the first end (22), and a length extending from the first end (22) to the second end (24), and is capable of rotating with respect to the revolving platform (30),
iii. the hollow tube (10) has an overlapping longitudinal seam area (16) having two plies of the packaging film and has a first end (12) corresponding to the first end (22) of the mandrel (20) and a second end (14) distal thereof, the second end (14) extending beyond the second end (24) of the mandrel (20) to define a protruding tube portion (18), and a length extending from the first end (12) to the second end (14); wherein the protruding tube portion (18) has a length approximately equal to the radius of the hollow tube (10);
b. forming a folded surface by rotating the hollow tube (10) and mandrel (20) while counter-rotating a plate (40) having a plurality of folding blades (42) extending outwardly therefrom, distal an axis of rotation thereof to form a plurality of folds that define the folded surface, substantially perpendicular to the longitudinal axis of the hollow tube (10), each folding blade (42) engaging a portion of the protruding tube portion (18);
c. applying heat and pressure to the folded surface to form a closed end;
d. inserting the tampon into the tube (10); and
e. closing the first end (12) of the tube.

2. The method of claim 1, wherein the packaging film comprises a thermoplastic film.

3. The method of claim 1, wherein the packaging film comprises a thermoplastic surface coating layer.

4. The method of claim 1, wherein the step of providing a packaging film on a mandrel (20) in the form of a hollow tube (10) comprises
a) winding a packaging film around a mandrel (20) to form a hollow tube (10), wherein:
i) the packaging film material comprises at least one thermoplastic surface;
ii) the mandrel (20) has a first end (22) extending from a revolving platform (30) and a second end (24), distal the first end (22), and a length extending from the first end (22) to the second end (24),
iii) the hollow tube (10) has an overlapping longitudinal seam area (16) having two plies of the packaging film and has a first end (12) corresponding to the first end (22) of the mandrel (20) and a second end (14) distal thereof, the second end (14) extending beyond the second end (24) of the mandrel (20) to define a protruding tube portion (18), and a length extending from the first end (12) to the second end (14); wherein the protruding tube portion (18) has a length approximately corresponding to the radius of the hollow tube (10);
b) applying heat and pressure to the overlapping longitudinal seam area (16) to form a longitudinal seam;

5. Apparatus for forming a package tube for a tampon comprising:
- a plurality of rotating mandrels, each mandrel (20) having a first end (22) extending from a revolving platform (30) and a second end (24) distal the first end (22); **characterized in that** the apparatus further comprises:
- a tube closing station comprising a plurality of folding blades (42) sequentially arranged and configured in a fan pattern in a plane perpendicular to an axis of rotation of the revolving platform (30) and a heater;
wherein each of the plurality of rotating mandrels are adapted to be indexed to the tube closing station and to rotate in a direction opposite of the folding blades (42) to form a series of folds in an end of a tube (10) of packaging film formed about such rotating mandrel (20) to thermally bond the series of folds on the second end (24) of each mandrel (20).

6. The apparatus of claim 5 wherein the folding blades (42) are separated with a curved leading edge (48) and sequentially arranged and configured in a fan pattern.

7. The apparatus of claim 5 wherein the folding blades (42) are formed in a wave-shaped plate.

## Patentansprüche

1. Verfahren zur Verpackung eines Tampons, umfassend die Schritte:
a. Bereitstellen einer Verpackungsfolie auf einem Formkern (20) in Form eines hohlen Rohrs (10), wobei:
i. das Verpackungsfolienmaterial mindestens eine thermoplastische Oberfläche umfasst;
ii. der Formkern (20) ein erstes Ende (22) aufweist, das sich von einer Drehplattform (30) erstreckt, und ein zweites Ende (24), distal zu dem ersten Ende (22), und eine Länge, die sich von dem ersten Ende (22) bis zu dem zweiten Ende (24) erstreckt, und sich mit Bezug auf die Drehplattform (30) drehen kann,
iii. das hohle Rohr (10) eine überlappende Längsnahtfläche (16) mit zwei Lagen der Verpackungsfolie aufweist und ein erstes Ende (12) aufweist, das dem ersten Ende (22) des Formkerns (20) entspricht, und ein zweites Ende (14) distal davon, wobei sich das zweite Ende (14) über das zweite Ende (24) des Formkerns (20) hinauserstreckt, um einen vorstehenden Rohrabschnitt (18) zu definieren, und eine Länge, die sich von dem ersten Ende (12) bis zu dem zweiten Ende (14) erstreckt, wobei der vorstehende Rohrabschnitt (18) eine Länge aufweist, die etwa dem Radius des hohlen Rohrs (10) entspricht;
b. Bilden einer gefalzten Oberfläche durch Drehen des hohlen Rohrs (10) und des Formkerns (20) während der Gegendrehung einer Platte (40), die eine Vielzahl von Falzmessern (42), die sich davon nach außen erstrecken, distal an der Drehachse davon aufweist, um eine Vielzahl von Falzen zu bilden, die die gefalzte Oberfläche definieren, im Wesentlichen senkrecht zu der Längsachse des hohlen Rohrs (10), wobei jedes Falzmesser (42) einen Abschnitt des vorstehenden Rohrabschnitts (18) in Eingriff nimmt;
c. Aufbringen von Wärme und Druck auf die gefalzte Oberfläche, um ein geschlossenes Ende zu bilden;
d. Einsetzen des Tampons in das Rohr (10); und
e. Verschließen des ersten Endes (12) des Rohrs.

2. Verfahren nach Anspruch 1, wobei die Verpackungsfolie eine thermoplastische Folie umfasst.

3. Verfahren nach Anspruch 1, wobei die Verpackungsfolie eine thermoplastische Oberflächenbeschichtungsschicht umfasst.

4. Verfahren nach Anspruch 1, wobei der Schritt des Bereitstellens einer Verpackungsfolie auf einem Formkern (20) in Form eines hohlen Rohrs (10) umfasst
a) Wickeln einer Verpackungsfolie um einen Formkern (20), um ein hohles Rohr (10) zu bilden, wobei:
i) das Verpackungsfolienmaterial mindestens eine thermoplastische Oberfläche umfasst;
ii) der Formkern (20) ein erstes Ende (22) aufweist, das sich von einer Drehplattform (30) erstreckt, und ein zweites Ende (24), distal zu dem ersten Ende (22), und eine Länge, die sich von dem ersten Ende (22) bis zu dem zweiten Ende (24) erstreckt,
iii) das hohle Rohr (10) eine überlappende Längsnahtfläche (16) mit zwei Lagen der Verpackungsfolie aufweist und ein erstes Ende (12) aufweist, das dem ersten Ende (22) des Formkerns (20) entspricht, und ein zweites Ende (14) distal davon, wobei sich das zweite Ende (14) über das zweite Ende (24) des Formkerns (20) hinauserstreckt, um einen vorstehenden Rohrabschnitt (18) zu definieren, und eine Länge, die sich von dem ersten Ende (12) bis zu dem zweiten Ende (14) erstreckt; wobei der vorstehende Rohrabschnitt (18) eine Länge aufweist, die etwa dem Radius des hohlen Rohrs (10) entspricht;
b) Aufbringen von Wärme und Druck auf die überlappende Längsnahtfläche (16), um eine Längsnaht zu bilden;

5. Vorrichtung zur Herstellung eines Verpackungsrohrs für einen Tampon, umfassend:
eine Vielzahl von Drehformkernen, wobei jeder Formkern (20) ein erstes Ende (22) aufweist, das sich von einer Drehplattform (30) erstreckt, und ein zweites Ende (24) distal zu dem ersten Ende (22); **dadurch gekennzeichnet, dass** die Vorrichtung ferner umfasst:
- eine Rohrverschließstation, die eine Vielzahl von Falzmessern (42) umfasst, die nacheinander angeordnet und fächerartig in einer Ebene senkrecht zu einer Drehachse der Drehplattform (30) und einer Heizeinrichtung ausgelegt sind;
wobei jeder der Vielzahl von Drehformkernen dazu angepasst ist, an der Rohrverschließstation indiziert zu werden und in eine Richtung entgegen der Falzmesser (42) zu drehen, um eine Reihe von Falzen in einem Ende eines Rohrs (10) der um so einen Drehformkern (20) gebildeten Verpackungsfolie zu bilden, um die Reihe von Falzen an dem zweiten Ende (24) eines jeden Formkerns (20) thermisch zu verbinden.

6. Vorrichtung nach Anspruch 5, wobei die Falzmesser (42) durch eine gekrümmte Vorderkante (48) getrennt und nacheinander angeordnet und in einem Fächermuster ausgelegt sind.

7. Vorrichtung nach Anspruch 5, wobei die Falzmesser (42) in einer wellenförmigen Platte gebildet sind.

## Revendications

1. Procédé d'emballage d'un tampon, comprenant les étapes suivantes :
a. fournir un film d'emballage sur un mandrin (20) sous la forme d'un tube creux (10),
i. le matériau du film d'emballage comprenant au moins une surface thermoplastique ;
ii. le mandrin (20) ayant une première extrémité (22) s'étendant depuis une plate-forme tournante (30) et une deuxième extrémité (24), distale par rapport à la première extrémité (22), et une longueur s'étendant depuis la première extrémité (22) jusqu'à la deuxième extrémité (24), et étant capable de tourner par rapport à la plate-forme tournante (30),
iii. le tube creux (10) a une zone de joint longitudinale de chevauchement (16) ayant deux couches de film d'emballage et ayant une première extrémité (12) correspondant à la première extrémité (22) du mandrin (20) et une deuxième extrémité (14) distale par rapport à celle-ci, la deuxième extrémité (14) s'étendant au-delà de la deuxième extrémité (24) du mandrin (20) pour définir une portion de tube saillante (18), et une longueur s'étendant depuis la première extrémité (12) jusqu'à la deuxième extrémité (14) ; la portion de tube saillante (18) ayant une longueur approximativement égale au rayon du tube creux (10) ;
b. former une surface pliée en faisant tourner le tube creux (10) et le mandrin (20) tout en faisant tourner dans le sens inverse une plaque (40) ayant une pluralité de lames de pliage (42) s'étendant vers l'extérieur depuis celle-ci, distalement par rapport à un axe de rotation de celle-ci pour former une pluralité de plis qui définissent la surface pliée, sensiblement perpendiculairement à l'axe longitudinal du tube creux (10), chaque lame de pliage (42) venant en prise avec une portion de la portion de tube saillante (18) ;
c. appliquer de la chaleur et de la pression à la surface pliée pour former une extrémité fermée ;
d. insérer le tampon dans le tube (10) ; et
e. fermer la première extrémité (12) du tube.

2. Procédé selon la revendication 1, dans lequel le film d'emballage comprend un film thermoplastique.

3. Procédé selon la revendication 1, dans lequel le film d'emballage comprend une couche de revêtement de surface thermoplastique.

4. Procédé selon la revendication 1, dans lequel l'étape de fourniture d'un film d'emballage sur un mandrin (20) sous la forme d'un tube creux (10) comprend
a) l'enroulement d'un film d'emballage autour d'un mandrin (20) pour former un tube creux (10),
i) le matériau de film d'emballage comprenant au moins une surface thermoplastique ;
ii) le mandrin (20) ayant une première extrémité (22) s'étendant depuis une plate-forme tournante (30) et une deuxième extrémité (24), distale par rapport à la première extrémité (22), et une longueur s'étendant depuis la première extrémité (22) jusqu'à la deuxième extrémité (24),
iii) le tube creux (10) ayant une zone de joint longitudinale de chevauchement (16) ayant deux couches de film d'emballage et ayant une première extrémité (12) correspondant à la première extrémité (22) du mandrin (20) et une deuxième extrémité (14) distale par rapport à celle-ci, la deuxième extrémité (14) s'étendant au-delà de la deuxième extrémité (24) du mandrin (20) pour définir une portion de tube saillante (18), et une longueur s'étendant depuis la première extrémité (12) jusqu'à la deuxième extrémité (14) ; la portion de tube saillante (18) ayant une longueur approximativement égale au rayon du tube creux (10) ;
b) appliquer de la chaleur et de la pression à la zone de joint longitudinale de chevauchement (16) pour former un joint longitudinal.

5. Appareil pour former un tube d'emballage pour un tampon, comprenant :
- une pluralité de mandrins rotatifs (20) ayant une première extrémité (22) s'étendant depuis une plate-forme tournante (30) et une deuxième extrémité (24) distale par rapport à la première extrémité (22) ;
**caractérisé en ce que** l'appareil comprend en outre :
- un poste de fermeture de tube comprenant une pluralité de lames de pliage (42) disposées séquentiellement et configurées suivant un motif en éventail dans un plan perpendiculaire à un axe de rotation de la plate-forme tournante (30) et un dispositif de chauffage ; chacun parmi la pluralité de mandrins rotatifs étant prévu pour être indexé au poste de fermeture de tube et pour tourner dans une direction opposée aux lames de pliage (42) pour former une série de plis dans une extrémité d'un tube (10) de film d'emballage formé autour d'un tel mandrin rotatif (20) afin de coller thermiquement la série de plis sur la deuxième extrémité (24) de chaque mandrin (20) .

6. Appareil selon la revendication 5, dans lequel les lames de pliage (42) sont séparées avec un bord avant courbe (48) et sont disposées séquentiellement et sont configurées suivant un motif en éventail.

7. Appareil selon la revendication 5, dans lequel les lames de pliage (42) sont formées en une plaque de forme ondulée.
